(19) Europäisches Patentamt
European Patent Office
Office européen des brevets

(11) Veröffentlichungsnummer: **0 022 996**
**B1**

(12) # EUROPÄISCHE PATENTSCHRIFT

(45) Veröffentlichungstag der Patentschrift: **25.04.84**

(51) Int. Cl.³: **A 61 K 7/09**, A 61 K 7/11

(21) Anmeldenummer: **80104014.8**

(22) Anmeldetag: **11.07.80**

(54) **Verfahren zur dauerhaften Haarverformung.**

(30) Priorität: **24.07.79 DE 2929865**

(43) Veröffentlichungstag der Anmeldung: **28.01.81 Patentblatt 81/4**

(45) Bekanntmachung des Hinweises auf die Patenterteilung: **25.04.84 Patentblatt 84/17**

(84) Benannte Vertragsstaaten: **AT DE GB IT**

(56) Entgegenhaltungen:
**DE - A - 2 262 940**
**FR - A - 1 043 145**
**FR - A - 1 268 548**
**FR - E - 80 815**
**GB - A - 773 559**
**GB - A - 1 158 346**
**GB - A - 1 198 857**

(73) Patentinhaber: **Wella Aktiengesellschaft, Berliner Allee 65, D-6100 Darmstadt (DE)**

(72) Erfinder: **Lang, Günther, Dr., Frankensteiner Strasse 1, D-6101 Niederbeerbach (DE)**
Erfinder: **Wajaroff, Theodor, Erbacher Strasse 56b, D-6100 Darmstadt (DE)**

**EP 0 022 996 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

# Verfahren zur dauerhaften Haarverformung

Die vorliegende Erfindung betrifft ein Verfahren zur dauerhaften Haarverformung, bei dem durch Verwendung von ungesättigten organischen Verbindungen eine allmähliche Herabsetzung der Konzentration der haarkeratinreduzierenden Substanz erfolgt und dadurch eine haarschonende Verformung ermöglicht wird.

Es ist bereits ein Verfahren zur Dauerverformung von Haaren bekannt, in dem ein Verformungsmittel mit einem Gehalt an einer haarkeratinreduzierenden Substanz sowie außerdem einer ungesättigten Verbindung, die eine Kohlenstoff-Kohlenstoff-Doppelbindung aufweist, verwendet wird. Die ungesättigte Verbindung soll geeignet sein, bei Anwesenheit der haarkeratinreduzierenden Substanz zu polymerisieren und langkettige Polymerisate im Haar zu bilden. Die wasserunlöslichen Polymerisate verleihen dem Haar ein stützendes Gerüst, welches die Haarfasern verstärkt. Der Verlust an Stärke, den das Haar durch die Spaltung der Disulfidbrückenbindungen erleidet, wird auf diese Weise wieder kompensiert (vgl. GB-PS 1 198 857).

Weiterhin ist schon ein Verfahren zur dauerhaften Verformung von Haaren bekanntgeworden, welches nur aus einem Arbeitsgang besteht und bei dem auf die sonst übliche Verfahrensstufe der Fixierung verzichtet wird. Die in diesem Verfahren verwendete Verformungslösung enthält als Verformungswirkstoff ein eine Thiolgruppe tragendes Reduktionsmittel im Gemisch mit einem Dimethacrylat des Glykols. Das Verfahren dient hauptsächlich dem Zweck, den Prozeß der Dauerverformung zu verkürzen und zu vereinfachen (FR-PS 1 268 548).

Schließlich ist auch ein Verfahren zur Dauerwellung von Haaren bekannt, bei dessen Anwendung man auf das gewickelte Haar zuerst ein Mittel, enthaltend eine für diesen Zweck bekannte keratinaufspaltende Substanz, einwirken läßt und anschließend mit einem Mittel behandelt, welches eine Substanz mit mindestens einer ungesättigten aliphatischen Kohlenstoff-Kohlenstoff-Bindung enthält. Die Verwendung der ungesättigten Substanz soll dem Ziel dienen, mit dem durch die vorangegangene Spaltung der Disulfidbrückenbindungen entstandenen Mercaptogruppen des Haarkeratins zu reagieren und dadurch eine bessere Festigung der Dauerwelle sowie eine leichte Handhabung und Kämmbarkeit des Haares zu bewirken. Ferner soll sie auch mit den nach der Einwirkung der keratinaufspaltenden Mercaptoverbindungen noch im Haar verbliebenen überschüssigen Resten reagieren und diese dadurch aus dem Haar beseitigen (GB-PS 773 559).

Gegenstand der vorliegenden Erfindung ist ein Verfahren zur dauerhaften Haarverformung, bei dem man ein Verformungsmittel, das zur allmählichen Herabsetzung der Konzentration der in diesem Mittel enthaltenen keratinreduzierenden Substanz mindestens eine organische Verbindung mit aktivierter aliphatischer Kohlenstoff-Kohlenstoff-Mehrfachbindung enthält, verwendet.

Die dauerhafte Haarverformung erfolgt bei den zur Zeit üblichen Verfahren in zwei Stufen. Zunächst werden durch die Einwirkung eines geeigneten Reduktionsmittels die Disulfidbrücken des Haarkeratins gespalten. Das Haar wird sodann in eine neue Form gebracht und anschließend durch die Behandlung mit einem geeigneten Oxidationsmittel, unter Wiederverknüpfung der gespaltenen Disulfidbindungen, in der neuen Form fixiert.

Die zur Durchführung der ersten, reduzierenden Verfahrensstufe verwendeten Mittel enthalten als verformungswirksame keratinreduzierende Substanz Sulfit oder bestimmte Mercaptoverbindungen, insbesondere Thioglykolsäure, Thiomilchsäure, auch in Form ihrer Salze mit anorganischen Basen oder auch Thioglycerin bzw. Derivate dieser Mercaptoverbindungen. In der Regel sind diese Mittel alkalisch eingestellt, um eine Haarerweichung und damit ein rasches Eindringen der keratinreduzierenden Substanz in das Haarkeratin zu erreichen.

Die erforderliche Alkalität wird hierbei vor allem durch die Zugabe von Ammoniak, organischen Aminen, Ammonium- und Alkalicarbonat, Ammonium- und Alkalihydrogencarbonat sowie Alkalihydroxiden erreicht.

Damit bei diesen Verfahren während der relativ kurzen Einwirkungszeit dieser Mittel auf das Haar eine ausreichende Haarverformung erzielt wird, müssen sowohl die verformungswirksame keratinreduzierende Substanz als auch die genannten alkalischen Bestandteile der Mittel in großem Überschuß eingesetzt werden. Diese sind während der gesamten Einwirkungszeit voll aktiv. Dabei nimmt ihre Wirksamkeit im Verlauf der Einwirkungszeit auf das allmählich erweichende Haar ständig zu.

Wird die beschriebene reduzierende Verfahrensstufe nicht rechtzeitig abgebrochen und das dabei verwendete Reduktionsmittel nicht gründlich entfernt, so besteht die Gefahr von Haarschädigungen, zum Beispiel durch Überkrausung, Haarbruch oder Haarausfall. Besonders gefährdet ist insbesondere ein durch eine zuvor erfolgte Dauerwell- oder Färbebehandlung chemisch geschädigtes Haar sowie dünnes Haar. Wegen der unterschiedlichen Haarbeschaffenheit muß deshalb zur Bestimmung der erforderlichen Einwirkungszeit dieser Mittel die erzielte Haarverformung ständig überprüft werden.

Es sind Verfahren zur Dauerverformung von Haaren bekannt, bei denen zur Vermeidung von Haarschädigungen während der reduzierenden Verfahrensstufe die Alkalität kontinuierlich erhöht wird. So ist in der deutschen Auslegeschrift 1 089 124 ein Verfahren beschrieben, bei dem das Alkalisierungsmittel Ammoniak erst durch die Zugabe von Urease zu einem Harnstoff enthaltenden

Verformungsmittel allmählich erzeugt wird. Die hohe Konzentration des verformungswirksamen keratinreduzierenden Bestandteils, nämlich der Mercaptoverbindung, in dem Verformungsmittel bleibt hierbei jedoch nahezu unverändert erhalten.

Es bestand daher die Aufgabe, ein Verfahren zur dauerhaften Haarverformung zu finden, das gegenüber den bekannten Verfahren eine haarschonendere Dauerverformung gestattet.

Diese Aufgabe wird durch das erfindungsgemäße Verfahren in hervorragendem Maße gelöst. Gegenstand der Erfindung ist daher ein Verfahren zur Dauerverformung von Haaren, bei dem man das Haar zuerst mit einem Verformungsmittel behandelt, in der gewünschten Form festhält, gegebenenfalls spült, sodann mit einem Fixiermittel nachbehandelt und spült, dadurch gekennzeichnet, daß man als Verformungsmittel eine aus zwei Komponenten erhaltene Mischung, deren Komponente 1 eine haarkeratinreduzierende Substanz enthält, während die Komponente 2 aus mindestens einer organischen Verbindung, die ausgewählt ist aus

I. Carbonsäuren mit aktivierter Doppelbindung der allgemeinen Formel

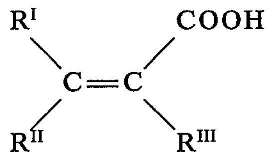

wobei die Substituenten $R^I$ und $R^{II}$ einen der Reste H, COOH, $CH_2COOH$, $CH(CH_3)COOH$, $C_6H_5$ oder $CH_3$, $R^{III}$ einen der Reste H, $C_1$- bis $C_6$-Alkyl, $CH_2COOH$ darstellen, unter der Voraussetzung daß $R^I$ verschieden von $R^{II}$ ist und nur einer der Reste $R^I$, $R^{II}$ und $R^{III}$ gleichzeitig $CH_2COOH$ bedeuten kann, sowie den Lithium-, Natrium-, Kalium- oder Ammonium-Salzen dieser Säuren, oder

II. Carbonsäuren mit aktivierter Dreifachbindung der Formel

$$HOOC \equiv C - R^{IV}$$

in der der Substituent $R^{IV}$ die Bedeutung H oder COOH hat, sowie den Lithium-, Natrium-, Kalium- oder Ammonium-Salzen dieser Säuren, besteht oder diese enthält, verwendet.

Die Komponente 1 des vorstehend genannten Verformungsmittels soll als haarkeratinreduzierende Substanz ein Sulfit, vorzugsweise ein Alkali- oder Ammoniumsulfit, ein Alkali- oder Ammoniumhydrogensulfit oder eine Mercaptoverbindung, insbesondere Thioglykolsäure oder Thiomilchsäure, auch in Form der Salze mit anorganischen oder organischen Basen, enthalten.

Bezogen auf die Gesamtmenge des Verformungsmittels soll darin die haarkeratinreduzierende Substanz in einem Anteil von etwa 1 bis 15 Gewichtsprozent, vorzugsweise 5 bis 12 Gewichtsprozent, enthalten sein.

Organische Verbindungen mit aktivierter aliphatischer Kohlenstoff-Kohlenstoff-Mehrfachbindung, die in dem für das erfindungsgemäße Verfahren zu verwendenden Verformungsmittel enthalten sein können, sind die aliphatischen Carbonsäuren

Aconitsäure
Acetylendicarbonsäure
Äthylmaleinsäure
α-Äthylcrotonsäure
i-Amylmaleinsäure
Angelicasäure
n-Butylfumarsäure
n- und i-Butylmaleinsäure
Citraconsäure
Crotonsäure
Fumarsäure
trans-Glutaconsäure
Isopropylmaleinsäure
Maleinsäure
Mesaconsäure
cis-β-Methylglutaconsäure
trans-α-Methylglutaconsäure
Propiolsäure
Zimtsäure

ferner die Salze dieser Carbonsäuren mit anorganischen oder organischen Basen, wobei als anorganische Basen die des Li, Na, K oder $NH_4$ in Betracht kommen.

Die organischen Verbindungen mit aktivierter aliphatischer Kohlenstoff-Kohlenstoff-Mehrfachbindung sollen, bezogen auf die Gesamtmenge des Verformungsmittels, darin in einer Menge von etwa 1 bis 20 Gewichtsprozent, vorzugsweise 1 bis 10 Gewichtsprozent, enthalten sein.

Die Komponente 2 des Verformungsmittels kann zur Beschleunigung der Oxidationswirkung des Luftsauerstoffs auf die keratinreduzierende Verbindung anorganische Redoxkatalysatoren, wie zum Beispiel Übergangsmetallsalze, insbesondere Salze des Mangans, Eisens und Kupfers, enthalten.

Das Verformungsmittel kann weiterhin organische Redoxindikatoren, wie z. B. Indigo, Thionin, Methylenblau und Indigocarmin, enthalten. Diese Indikatoren lassen durch ihre Farbe unmittelbar eine eventuell eingetretene Unbrauchbarkeit des Reduktionsmittels erkennen.

Die Redoxkatalysatoren können in dem Verformungsmittel in einer Menge von etwa 0,001 bis 0,1 Gewichtsprozent enthalten sein, während die Redoxindikatoren in einer Menge von etwa 0,005 bis 0,02 Gewichtsprozent vorliegen können.

Das Verformungsmittel kann außerdem in Haarverformungsmitteln übliche Zusätze, wie zum Beispiel Alkalisierungsmittel, insbesondere Ammoniak, organische Amine, Ammonium- und Alkalicarbonat, Ammonium- und Alkalihydrogencarbonat sowie Alkalihydroxid, Netzmittel, Lösungsvermittler, Emulgatoren, Verdicker, Quellmittel, Füllstoffe, Farbstoffe, Parfümöle und andere, enthalten.

Die Zubereitungsform der Komponente 1 dieses Verformungsmittels kann die einer Lösung, eines Gels, einer Creme oder einer Emulsion sein, während die Komponente 2 darüber hinaus auch in fester Form, zum Beispiel als Pulver, Granulat oder Tablette, vorliegen kann.

Je nach Zusammensetzung kann das bei dem erfindungsgemäßen Verfahren verwendete Verformungsmittel sowohl schwach sauer als auch alkalisch reagieren.

Bei dem erfindungsgemäßen Verfahren zur Dauerverformung von Haaren vermischt man unmittelbar vor dem Gebrauch die beiden Komponenten des vorstehend beschriebenen Verformungsmittels miteinander, bringt das so erhaltene gebrauchsfertige Verformungsmittel gleichmäßig auf die Haare auf und hält das Haar in der gewünschten Form fest (zum Beispiel durch Aufwickeln der Haare auf Wickler oder durch Strecken mit einem Kamm oder einer Bürste). Nach einer Einwirkungszeit von etwa 10 bis 50 Minuten bei einer Temperatur von 20 bis 80°C wird das restliche Verformungsmittel gegebenenfalls mit Wasser ausgespült. Anschließend fixiert man das Haar durch Behandlung mit einem üblichen Fixiermittel, wie z. B. einer 2%igen wäßrigen Wasserstoffperoxidlösung, in der neuen Form und spült es schließlich mit Wasser gründlich nach.

Die haarschonende Dauerverformung bei Anwendung des erfindungsgemäßen Verfahrens wird durch folgenden Vorgang ermöglicht:

Mit dem Vermischen der beiden Komponenten des verwendeten Verformungsmittels unmittelbar vor dem Gebrauch wird die keratinreduzierende Verbindung x allmählich durch Addition an die aktivierte Mehrfachbindung der ungesättigten aliphatischen Verbindung y gemäß den nachstehenden beispielhaften Reaktionsgleichungen gebunden und für die Haarverformung unwirksam gemacht.

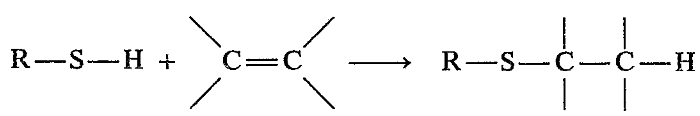

x y (R = Molekülrest der Mercaptoverbindung)

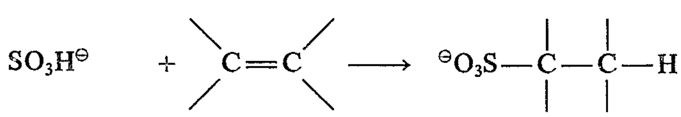

x y

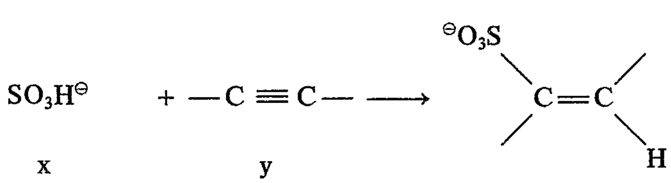

x y

Hierdurch läßt sich die Konzentration der keratinereduzierenden verformungswirksamen Verbindung zeitlich verändern und durch Wahl von Art und Menge der beiden Reaktanden x und y so einstellen, daß die zu Beginn der Einwirkungszeit des Verformungsmittels auf das Haar notwendige hohe Konzentration der keratinreduzierenden Verbindung x sich während der Einwirkungszeit mit gewünschter Geschwindigkeit verringert und so dem allmählich erweichenden Haar anpaßt.

Die nachstehenden Beispiele sollen den Gegenstand der Erfindung näher erläutern.

## Beispiele

### Beispiel 1

Eine Dauerwellösung der Zusammensetzung

Komponente 1
10,0 g Thioglykolsäure
8,0 g Ammoniak, 25%ig
6,1 g Ammoniumhydrogencarbonat
75,9 g Wasser
100,0 g

weist einen pH-Wert von 8,2 auf und enthält 10 Gewichtsprozent Thioglykolsäure. Dieses Dauerwellmittel würde bei der Anwendung in einem üblichen Dauerwellverfahren seine Wirksamkeit nahezu unverändert beibehalten. Wird jedoch nach dem erfindungsgemäßen Verfahren dieser Lösung (Komponente 1) kurz vor dem Gebrauch ein Pulver (Komponente 2) der nachstehenden Zusammensetzung

Komponente 2
7,6865 g Dilithiumfumarat
0,0085 g $MnSO_4 \cdot H_2O$
0,0050 g Indigocarmin
7,7000 g

zugegeben und durch Schütteln darin gelöst, so verringert sich der Thioglykolsäuregehalt des erhaltenen Verformungsmittels bei einer Temperatur von 30° C innerhalb von 20 Minuten um 25% auf 7,5 Gewichtsprozent und innerhalb von 40 Minuten um 40% auf 6 Gewichtsprozent, bezogen jeweils auf den Anfangsgehalt an Thioglykolsäure in Komponente 1.

Die unmittelbar nach dem Vermischen der beiden Komponenten durch den Indikator Indigocarmin hervorgerufene blaue Farbe des Verformungsmittels schlägt nach kurzer Zeit nach gelb um und zeigt so die Aktivität des keratinreduzierenden Bestandteils, nämlich der Thioglykolsäure, an. Wäre die Komponente 1, beispielsweise durch Offenstehen oder Beschädigung des Behältnisses, nicht mehr funktionsfähig, so würde dieser Farbumschlag nicht erfolgen.

Bei dem erfindungsgemäßen Verfahren trägt man das vorstehend genannte Verformungsmittel unmittelbar nach dem Erhalt gleichmäßig auf die gewickelten Haare auf und läßt es etwa 15 bis 30 Minuten einwirken. Danach wird das Haar mit Wasser gespült und in bekannter Weise oxidativ nachbehandelt. Schließlich werden die Wickler entfernt und das Haar gründlich mit Wasser ausgespült.

Es wird ein gutes Wellergebnis festgestellt, wobei die Gefahr von Haarschädigungen und Hautreizungen wesentlich verringert ist.

### Beispiel 2

Eine gute Dauerwellung von Haaren unter schonenden Bedingungen wird erzielt, indem man eine Lösung der Zusammensetzung

Komponente 1
10,90 g Ammoniumthioglykolat, 50%ige wäßrige Lösung
3,60 g Ammoniak, 25%ig
0,40 g Octylphenol, mit 20 Mol Äthylenoxid oxäthyliert
0,01 g 5,5'-Indigodisulfonsäure, Natriumsalz (Indigocarmin)
0,40 g Parfümöl
34,69 g Wasser
50,00 g

mit einem pH-Wert von pH = 9,5 und eine Lösung der Zusammensetzung

Komponente 2
3,700 g Zimtsäure
0,008 g $MnSO_4 \cdot H_2O$
1,700 g Ammoniak, 25%ig
44,592 g Wasser
50,000 g

mit einem pH-Wert von ebenfalls pH = 9,5, miteinander vermischt, das Haar mit dem so erhaltenen gebrauchsfertigen Verformungsmittel vorfeuchtet, sodann auf Wickler wickelt und die Wickler mit auf eine Temperatur von 80°C vorgeheizten Heizklammern versieht. Nach 15 Minuten Einwirkungszeit werden die Heizklammern entfernt und das Haar anschließend in bekannter Weise oxidativ fixiert. Nach dem Entfernen der Wickler wird das Haar gründlich mit Wasser ausgespült.

## Beispiel 3

Unmittelbar vor Beginn des Dauerwellverfahrens vermischt man die Komponenten 1 und 2 der nachstehend angegebenen Zusammensetzung miteinander.

Komponente 1

| | |
|---|---|
| 23,00 g | Monoäthanolaminsalz der Thioglykolsäure, 50%ige wäßrige Lösung |
| 1,80 g | Monoäthanolamin |
| 0,80 g | Octylphenol, mit 20 Mol Äthylenoxid oxäthyliert |
| 0,01 g | 5,5'-Indigodisulfonsäure, Natriumsalz (Indigocarmin) |
| 0,60 g | Parfümöl |
| 23,79 g | Wasser |
| 50,00 g | |

Der pH-Wert dieser Lösung beträgt 9,0.

Komponente 2

| | |
|---|---|
| 6,400 g | Crotonsäure |
| 0,003 g | $FeSO_4 \cdot 7\ H_2O$ |
| 5,100 g | Ammoniak, 25%ig |
| 38,497 g | Wasser |
| 50,000 g | |

Der pH-Wert dieser Lösung beträgt 7,0.

Es wird ein gebrauchsfertiges Verformungsmittel erhalten, das einen pH-Wert von pH = 9,0 aufweist. Dieses Verformungsmittel wird auf das gewickelte Haar aufgetragen und die Haare mit einer Kunststoffhaube abgedeckt. Anschließend führt man den Haaren mittels einer Trockenhaube Wärme zu und läßt das Verformungsmittel etwa 15 Minuten lang bei einer Temperatur von 45°C auf das Haar einwirken. Anschließend wird in üblicher Weise oxidativ fixiert. Nach dem Entfernen der Wickler spült man das Haar gründlich mit Wasser aus.

## Beispiel 4

Zur Durchführung einer Dauerwellbehandlung wird eine Lösung der Zusammensetzung

Komponente 1

| | |
|---|---|
| 22,8 g | Ammoniumsulfit, 35%ig |
| 19,6 g | Schwefelige Säure, 5%ig |
| 37,6 g | Wasser |
| 80,0 g | |

mit einer Lösung der Zusammensetzung

Komponente 2

| | |
|---|---|
| 8,000 g | Citraconsäure |
| 0,006 g | $FeSO_4 \cdot 7\ H_2O$ |
| 11,994 g | Wasser |
| 20,000 g | |

die zuvor mit Ammoniak auf einen pH-Wert von pH = 6,7 eingestellt wurde, vermischt. Man erhält ein gebrauchsfertiges Verformungsmittel. Dieses Verformungsmittel wird gleichmäßig auf das auf Lockenwickler gewickelte Haar aufgetragen.

Wird das Verformungsmittel unter weitgehendem Ausschluß von Luftsauerstoff (Haubenwelle) angewendet, so vermindert sich der Sulfitgehalt bei einer Temperatur von 45°C nach 30 Minuten Einwirkungszeit auf 88% des Ausgangswertes und nach 60 Minuten Einwirkungszeit auf 78% des Ausgangswertes.

Wird das Verformungsmittel bei freiem Zutritt von Luftsauerstoff und bei Raumtemperatur

(Kaltwelle) angewendet, so verringert sich der Sulfitgehalt bereits nach 20 Minuten Einwirkungszeit auf 50% des Ausgangswertes.

Die für eine Behandlung jeweils erforderliche Einwirkungszeit wird hierbei anhand eines Probewicklers bestimmt.

Schließlich wird das Haar mit Wasser gespült, wie üblich oxidativ fixiert, und die Wickler werden entfernt. Abschließend spült man das Haar gründlich mit Wasser aus.

## Beispiel 5

Eine Haarentkräuselung unter schonenden Bedingungen wird durchgeführt, indem man wie folgt verfährt:

Durch gründliches Vermischen einer Creme der Zusammensetzung

Komponente 1

14,24 g Ammoniumthioglykolat, 50%ige wäßrige Lösung
4,48 g Ammoniak, 25%ig
4,32 g Cetylalkohol
1,44 g Paraffinöl
3,12 g Oleylalkohol, mit 20 Mol Äthylenoxid oxäthyliert
1,20 g kolloidale Kieselsäure
0,24 g Parfümöl
50,96 g Wasser
80,00 g

deren pH-Wert 9,5 ist, mit einer Lösung der Zusammensetzung

Komponente 2

3,714 g Maleinsäure
0,003 g $CuSO_4$, wasserfrei
2,180 g Ammoniak, 25%ig
0,010 g 3,9-Bisdimethylaminophenazothioniumchlorid (Methylenblau)
14,093 g Wasser
20,000 g

deren pH-Wert mit Ammoniak auf pH=8 eingestellt wird, erhält man ein gebrauchsfertiges cremeförmiges Verformungsmittel, dessen Thioglykolsäuregehalt sich innerhalb von 5 Minuten auf 80%, und innerhalb von 10 Minuten auf 68% des ursprünglich vorhandenen Wertes verringert.

Das erhaltene cremeförmige Verformungsmittel wird sofort gleichmäßig auf das zu entkräuselnde Haar aufgetragen. Man kämmt das Haar, bis die gewünschte Entkräuselung eingetreten ist, spült gründlich mit Wasser aus und fixiert in üblicher Weise oxidativ. Abschließend wird das Haar mit Wasser gespült.

Durch dieses Verfahren ist eine gute Haarentkräuselung unter weitgehender Vermeidung von Haut- und Haarschädigungen möglich.

## Beispiel 6

Zur Entkräuselung von Haaren unter schonenden Bedingungen vermischt man eine Creme der Zusammensetzung

Komponente 1

12,40 g Ammoniumthioglykolat, 50%ige wäßrige Lösung
3,30 g Ammoniumhydrogencarbonat
3,90 g Oleylalkohol, mit 20 Mol Äthylenoxid oxäthyliert
1,50 g kolloidale Kieselsäure
1,80 g Paraffinöl
5,40 g Cetylalkohol
0,30 g Parfümöl
61,40 g Wasser
90,00 g

deren pH-Wert 8,2 beträgt, mit einer Lösung der Zusammensetzung

Komponente 2

| | |
|---|---|
| 1,6 g | Ammoniumpropiolat |
| 8,4 g | Wasser |
| 10,0 g | |

deren pH-Wert zuvor mit Ammoniak auf pH = 7 eingestellt wird, und erhält hierbei ein Verformungsmittel in Cremeform. Gegebenenfalls kann die Komponente 2 auch lediglich aus 1,6 g Ammoniumpropiolat bestehen und somit in Pulverform vorliegen.

Man trägt das so erhaltene Verformungsmittel sofort auf das gekrauste Haar auf und kämmt das Haar von Zeit zu Zeit glatt, bis die gewünschte Entkräuselung eingetreten ist. Danach wird das Haar mit Wasser ausgespült und wie üblich oxidativ fixiert. Anschließend wird das Haar mit Wasser gespült.

Alle in der vorliegenden Anmeldung angegebenen Prozentzahlen stellen Gewichtsprozente dar.

## Patentansprüche

1. Verfahren zur Dauerverformung von Haaren, bei dem man das Haar zuerst mit einem Verformungsmittel behandelt, in der gewünschten Form festhält, gegebenenfalls spült, sodann mit einem Fixiermittel nachbehandelt und spült, dadurch gekennzeichnet, daß man als Verformungsmittel eine aus zwei Komponenten erhaltene Mischung, deren Komponente 1 eine haarkeratinreduzierende Substanz enthält, während die Komponente 2 aus mindestens einer organischen Verbindung, die ausgewählt ist aus

I. Carbonsäuren mit aktivierter Doppelbindung der allgemeinen Formel

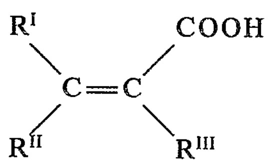

wobei die Substituenten $R^I$ und $R^{II}$ einen der Reste H, COOH, $CH_2COOH$, $CH(CH_3)COOH$, $C_6H_5$ oder $CH_3$, $R^{III}$ einen der Reste H, $C_1$- bis $C_6$-Alkyl, $CH_2COOH$ darstellen, unter der Voraussetzung, daß $R^I$ verschieden von $R^{II}$ ist und nur einer der Reste $R^I$, $R^{II}$ und $R^{III}$ gleichzeitig $CH_2COOH$ bedeuten kann, sowie den Lithium-, Natrium-, Kalium- oder Ammonium-Salzen dieser Säuren, oder

II. Carbonsäuren mit aktivierter Dreifachbindung der Formel

$$HOOC \equiv C - R^{IV}$$

in der der Substituent $R^{IV}$ die Bedeutung H oder COOH hat, sowie den Lithium-, Natrium-, Kalium- oder Ammonium-Salzen dieser Säuren, besteht oder diese enthält, verwendet.

2. Verfahren nach Anspruch 1, dadurch gekennzeichnet, daß man als Komponente 1 eine Zubereitung mit einem Gehalt an einem Sulfit, vorzugsweise einem Alkali- oder Ammoniumsulfit, oder einer Mercaptoverbindung, insbesondere Thioglykolsäure oder Thiomilchsäure, auch in Form der Salze mit anorganischen oder organischen Basen, verwendet.

3. Verfahren nach Anspruch 1 und 2, dadurch gekennzeichnet, daß man als Verformungsmittel eine Mischung mit einem Gehalt an 1 bis 15 Gewichtsprozent, vorzugsweise 5 bis 12 Gewichtsprozent, einer haarkeratinreduzierenden Substanz verwendet.

4. Verfahren nach Anspruch 1 bis 3, dadurch gekennzeichnet, daß man als Komponente 2 mindestens eine der ungesättigten aliphatischen Carbonsäuren Citraconsäure, Crotonsäure, Fumarsäure, Maleinsäure, Propiolsäure und Zimtsäure, auch in Form ihrer Salze mit organischen oder anorganischen Basen (die des Li, Na, K oder $NH_4$), oder eine Zubereitung mit einem Gehalt an mindestens einer dieser Carbonsäuren bzw. Salze verwendet.

5. Verfahren nach Anspruch 1 bis 4, dadurch gekennzeichnet, daß man als Verformungsmittel eine Mischung, die einen Gehalt an organischen Verbindungen mit aktivierter aliphatischer Kohlenstoff-Kohlenstoff-Mehrfachbindung gemäß Anspruch 1,I und/oder II in einer Menge von 1 bis 20 Gewichtsprozent, vorzugsweise 1 bis 10 Gewichtsprozent, aufweist, verwendet.

6. Verfahren nach den Ansprüchen 1 bis 5, dadurch gekennzeichnet, daß man als Komponente 2 eine Zubereitung mit einem Gehalt an einem anorganischen Redoxkatalysator, vorzugsweise an einem Salz der Übergangsmetalle Mangan, Eisen oder Kupfer, verwendet.

7. Verfahren nach den Ansprüchen 1 bis 6, dadurch gekennzeichnet, daß man als eine der beiden Komponenten eine Zubereitung mit einem Gehalt an einem organischen Redoxindikator, vorzugsweise Indigo, Thionin, Methylenblau oder Indigocarmin, verwendet.

## Claims

1. Process for the permanent shaping of hair, in which the hair is first treated with a shaping medium, held fixed in the desired form, optionally rinsed, then treated with a fixing medium and rinsed, characterised in that there is used as the shaping medium a mixture obtained from two components, of which component 1 contains a hair keratin reducing substance, whilst component 2 consists of or contains at least one organic compound, which is selected from:

I) carboxylic acids with activated double bonds of the general formula

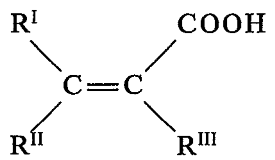

in which the substituents $R^I$ and $R^{II}$ represent one of the residues H, COOH, $CH_2COOH$, $CH(CH_3)COOH$, $C_6H_5$ or $CH_3$, $R^{III}$ represents one of the residues H, $C_1$- to $C_6$-alkyl, $CH_2COOH$, provided that $R^I$ is different from $R^{II}$ and only of the residues $R^I$, $R^{II}$ and $R^{III}$ can be $CH_2COOH$ at the same time, as well as the lithium, sodium, potassium or ammonium salts of these acids, or

II) carboxylic acids with activated triple bonds of the formula

$$HOOC \equiv C - R^{IV}$$

in which the substituent $R^{IV}$ has the meaning H or COOH, as well as the lithium, sodium, potassium or ammonium salts of such acids.

2. Process according to claim 1, characterised in that there is used as component 1 a preparation with a content of a sulphite, preferably an alkali metal or ammonium sulphite, or a mercapto-compound, especially thioglycolic acid or thiolactic acid, which may also be in the form of salts with inorganic or organic bases.

3. Process according to claim 1 and 2, characterised in that there is used as the shaping medium a mixture with a content of 1 to 15% by weight, preferably 5 to 12% by weight, of a hair keratin-reducing substance.

4. Process according to any of claims 1 to 3, characterised in that there is used as component 2 at least one of the unsaturated aliphatic carboxylic acid citraconic acid, crotonic acid, fumaric acid, maleic acid, propiolic acid, or cinnamic acid, which may also be in the form of their salts with organic or inorganic lithium, sodium, potassium, or ammonium bases, or a preparation with a content of at least one of these carboxylic acids or salts.

5. Process according to any of claims 1 to 4, characterised in that there is used as the shaping medium a mixture which has a content of organic compounds with activated aliphatic carbon-to-carbon multiple bonds according to claim 1, I and/or II, in an amount from 1 to 20% by weight, preferably 1 to 10% by weight.

6. Process according to any of claims 1 to 5, characterised in that there is used as component 2 a preparation with a content of an inorganic redox catalyst, preferably a salt of the transition metals manganese, iron, or copper.

7. Process according to any of claims 1 to 6, characterised in that there is used as one of the two components a preparation with a content of an organic redox indicator, preferably indigo, thionine, methylene blue, or indigo carmine.

## Revendications

1. Procédé pour la deformation permanente des cheveux, dans lequel on traite d'abord le cheveu avec un produit de deformation, on le maintient dans la forme recherchée, le cas échéant on le rince, puis on le traite avec un produit de fixation et on le rince, caractérisé par le fait qu'on utilise comme produit de deformation un mélange obtenu à partir de deux composants, dont le composant 1 contient une substance réduisant la kératine du cheveu, tandis que le composant 2 est constitué par au moins un composé organique, choisi parmi des

I) acides carboxyliques à liaison double activée de la formule générale

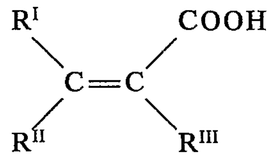

dans laquelle les groupes substituants $R^I$ et $R^{II}$ représentent un des radicaux H, COOH, $CH_2COOH$, $CH(CH_3)COOH$, $C_6H_5$ ou $CH_3$, $R^{III}$ est un des radicaux H, $C_1$- à $C_6$-alkyle, $CH_2COOH$, à la condition que $R^I$ soit différent de $R^{II}$ et que seul un des radicaux $R^I$, $R^{II}$, et $R^{III}$ puisse être en même temps $CH_2COOH$, ainsi que les sels de lithium, de sodium, de potassium ou d'ammonium de ces acides, ou

II) acides carboxyliques à triple liaison activée de la formule

$$HOOC \equiv C - R^{IV}$$

dans laquelle le groupe substituant $R^{IV}$ a la signification H ou COOH, ainsi que les sels de lithium, de sodium, de potassium ou d'ammonium de ces acides.

2. Procédé selon la revendication 1, caractérisé par le fait qu'on utilise comme composant 1 une préparation contenant un sulfite, de préférence un sulfite alcalin ou un sulfite d'ammonium, ou un composé mercaptan, notamment un acide thioglycolique ou un acide thiolactique, également sous la forme de leurs sels avec des bases inorganiques ou organiques.

3. Procédé selon la revendication 1 ou 2, caractérisé par le fait qu'on utilise comme produit de deformation un mélange ayant une teneur de 1 à 15% en poids, de préférence 5 à 12% en poids, d'une substance réduisant la kératine du cheveu.

4. Procédé selon les revendications 1 à 3, caractérisé par le fait qu'on utilise comme composant 2 ou moins un des acides carboxyliques aliphatiques non saturés suivants: citraconique, crotonique, fumarique, maléique, propiolique, ou cinnamique, également sous la forme de leurs sels avec des bases organiques ou non organiques, ceux du lithium, du sodium, du potassium ou de l'ammonium, ou bien une préparation contenant au moins un de ces acides carboxyliques ou de ces sels.

5. Procédé selon les revendications 1 à 4, caractérisé par le fait qu'on emploie comme produit de deformation un mélange comportant une teneur en composés organiques à liaisons multiples carbone-carbone aliphatiques activés selon la revendication 1, I et/ou II, de 1 à 20% en poids, de préférence 1 à 10% en poids.

6. Procédé selon les revendications 1 à 5, caractérisé par le fait qu'on utilise comme composant 2 une préparation contenant un catalyseur redox inorganique, de préférence un sel des métaux transistoires, manganèse, fer ou cuivre.

7. Procédé selon les revendications 1 à 6, caractérisé par le fait qu'on utilise comme l'un des deux composants une préparation contenant un indicateur redox organique, de préférence indigo, thionine, bleu de méthylène, carmin d'indigo.